# EUROPEAN PATENT SPECIFICATION

(11) **EP 3 405 194 B1**
(45) Date of publication and mention of the grant of the patent: **21.05.2025**
(21) Application number: 17700554.3
(22) Date of filing: 18.01.2017
(51) Int. Cl.: A61K 45/06, A61K 31/428, A61P 35/00, A61K 31/282

(54) **A TEMPORARY INHIBITOR OF P53 IN COMBINATION WITH AN ANTICANCER AGENT FOR USE IN THE PREVENTING OR REDUCING CANCER RELAPSE**
EIN TEMPORÄRER P53-HEMMER IN KOMBINATION MIT EINEM ANTI-KREBS MITTEL ZUR VERWENDUNG IN DER VERHINDERUNG ODER REDUZIERUNG EINES KREBSRÜCKFALLS
UN INHIBITEUR TEMPORAIRE DE P53 EN COMBINAISON AVEC UN AGENT ANTI-CANCEREUX ET LEUR UTILISATION À DES FINS DE PRÉVENTION OU DE RÉDUCTION DES RECHUTES DE CANCER

(30) Priority: 18.01.2016 EP 16305046
(43) Date of publication of application: 28.11.2018
(73) Proprietor: Institut National de la Santé et de la Recherche Médicale (INSERM), 75013 Paris (FR); Université de Montpellier, 34090 Montpellier (FR)
(72) Inventor: PUEL, Jean-Luc, 34295 Montpellier Cedex 5 (FR); MENARDO, Julien, 66000 Perpignan (FR); WANG, Jing, 34295 Montpellier Cedex 5 (FR); BENKAFADAR, Nesrine, 34295 Montpellier Cedex 5 (FR)
(74) Representative: Cabinet Beau de Loménie
(86) International application number: PCT/EP2017/050971
(87) International publication number: WO 2017/125429

(56) References cited:
- WO-A2-00/44364
- WO-A2-2012/033525

## Description

### Field of the invention

The present invention relates to the field of cancer therapeutic treatments, and more precisely to therapeutic treatments aimed at preventing or reducing cancer relapse.

### Background of the invention

Current cancer treatments ultimately fail owing to metastasis and relapse. Although chemotherapy can induce partial or even complete cancer regression in some patients, such initial responses are invariably followed by relapse, with the recurrent cancer being highly resistant to further chemotherapy, resulting in very limited survival benefits.

Notably, breast cancer (BCa) is the most common cancer diagnosis in women and the second-leading cause of cancer-related death among women (Ries LAG, et al. (eds). SEER Cancer Statistics Review, 1975-2003, National Cancer Institute, Bethesda, MD). Major advances in breast cancer treatment over the last 20 years have led to significant improvement in the rate of disease-free survival (DFS). For example, therapies utilizing antibodies reactive against tumor-related antigens have been used to block specific cellular processes in order to slow disease progress or prevent disease recurrence. Despite the recent advances in breast cancer treatment, a significant number of patients will ultimately die from recurrent disease.

Subpopulations of cancer cells with extremely high tumorigenic potential, termed cancer stem cells or stem-like cancer cells, have been isolated from patients with a variety of tumor types and have been found to possess high stemness properties. It has been demonstrated that these stemness-high malignant cells are extremely tumorigenic and are resistant to conventional chemotherapies and radiation. Moreover, it has been shown that chemotherapy and radiation induce stemness genes in cancer cells, thus converting stemness-low cancer cells to stemness-high cancer cells. Such highly tumorigenic and drug-resistant stemness-high cancer stem cells are likely to contribute to cancer relapse. Thus, there is a need for treatments that prevent or slow or prohibit the development of cancer disease relapse or recurrence, which encompasses breast cancer relapse. There is notably a need for substances able to prevent or slow or prohibit the development of cancer disease relapse or recurrence by killing cancer stem cells, including for substances that exert these properties when they are used in combination with other active agents or treatments, which other active agents or treatments encompass anti-cancer agents and anti-cancer treatments.

### Summary of the invention

The references to methods of treatment in the subsequent paragraphs of this description are to be interpreted as references to the compounds, pharmaceutical compositions and medicaments of the present invention for use in a method for treatment of the human (or animal) body by therapy.

The present invention relates to a temporary inhibitor of p53 for use for preventing or reducing cancer relapse in cancer patients, wherein the temporary inhibitor of p53 is used in combination with an anti-cancer treatment, said anti-cancer treatment being a radiation or an anti-cancer agent, and wherein the temporary inhibitor of p53 is chosen from pifithrin-alpha, pifithrin-µ, and cyclic pifithrin-alpha hydrobromide.

In some embodiments, the said temporary inhibitor of p53 and the said anti-cancer agent are each separately administered to the said cancer patient.

In some embodiments, the said temporary inhibitor of p53 is administered previously to the said anti-cancer agent.

In some embodiments, the administration of the said temporary inhibitor of p53 to the said cancer patient is stopped previously to stopping the administration of the said anti-cancer agent.

In some embodiments, the said temporary inhibitor of p53 and the said anti-cancer agent are concomitantly administered to the said cancer patient.

In some embodiments, the said temporary inhibitor of p53 and the said anti-cancer agent are combined in a single pharmaceutical composition which is administered to the said cancer patient.

The present invention also pertains to an anti-cancer agent for use in combination with a temporary inhibitor of p53 for use for preventing or reducing cancer relapse in cancer patients wherein the temporary inhibitor of p53 is chosen from pifithrin-alpha, pifithrin -µ, and cyclic pifithrin-alpha hydrobromide. In some embodiments, the said anticancer agent is selected in a group comprising cisplatin, carboplatin, oxaliplatie, nédaplatin, satraplatin, picoplatin, phenanthriplatin, triplatin, lipoplatin, Pt(ESDT)(Py)Cl, Methotrexate, Doxorubicin, Daunorubicin, Temozolomide (TMZ) and Chloroethylating nitrosoureas.

In some embodiments, the said anticancer agent is cisplatin or a cisplatin derivative or a cisplatin complex or a DNA-damaging compound.

In some embodiments, the said cancer patients are affected with breast cancer, somatic TP53 mutation induced cancers including: Ovarian, esophageal, colorectal, bladder, head and neck, cervical , larynx, lung and testicule cancers; and TP53 germline mutation induced early onset cancers such sarcomas, brain tumors, and adrenal cortical carcinomas. Specific embodiments of the present invention are disclosed in the claims.

### Description of the figures

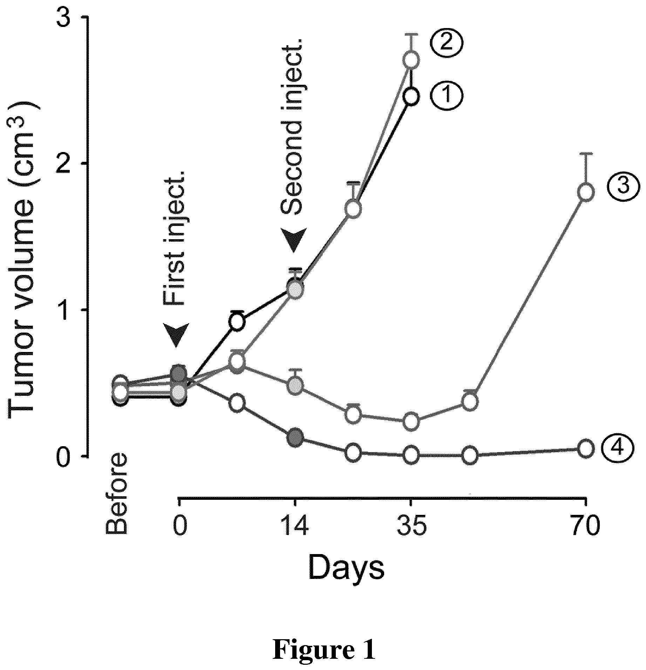

**Figure 1****:** Results of dissected tumors collected on day 35 and tumor growth curves. Weekly measurements were performed to determine tumor volume. Note the complete disappearance of tumor at day 35 and significantly reduced tumor regrowth up to day 70 in CDDP+ PFT-α treated mice. Curve n°1: DMSO. Curve n°2: PFT-α alone. Curve n°3: CDDP. Curve n°4: combination of CDDP and PFT-α. Abscissa: number of days after injection of the indicated compound. Ordinate; tumor volume, as expressed in cm³.

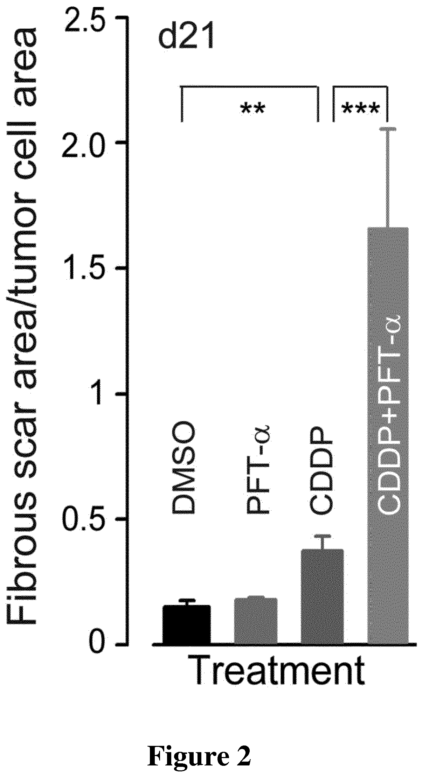

**Figure 2****:** Ratio of fibrous scar area/tumor cell area (sum of fibrous scar area/ sum of tumor cell area). Note that combined treatments were much more efficient in replacing tumor cells by fibrous scar. Abscissa; from left to right of the figure, DMSO, PFT-alpha, CDDP, combination of CDDP and PFT-alpha, respectively. Ordinate: values of the ratio fibrous scar area / tumor cell area.

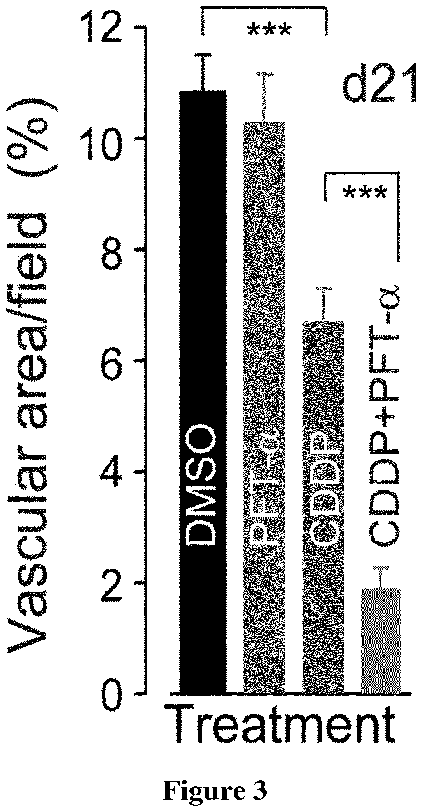

**Figure 3****:** Histograms representing the percentage of vascular area calculated using 2D reconstruction image analysis and the formula: vascular area = area of CD31-positive objects + lumen area per field area × 100%. The tumors from the different treated groups were collected at day 21. Abscissa; from left to right of the figure, DMSO, PFT-alpha, CDDP, combination of CDDP and PFT-alpha, respectively. Ordinate :percentage values of the vascular area / field.

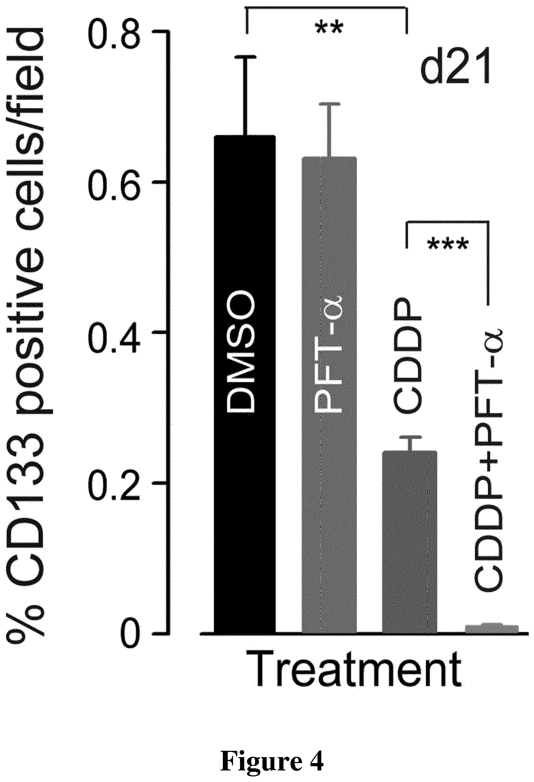

**Figure 4****:** Histograms representing the percentage of CD133 positive cells per field (% of CD-133 positive cells per total Hoechst 33342 labelled nuclei per field). The tumors were collected at day 21. Abscissa; from left to right of the figure, DMSO, PFT-alpha, CDDP, combination of CDDP and PFT-alpha, respectively. Ordinate: percentage of CD133 positive cells per field.

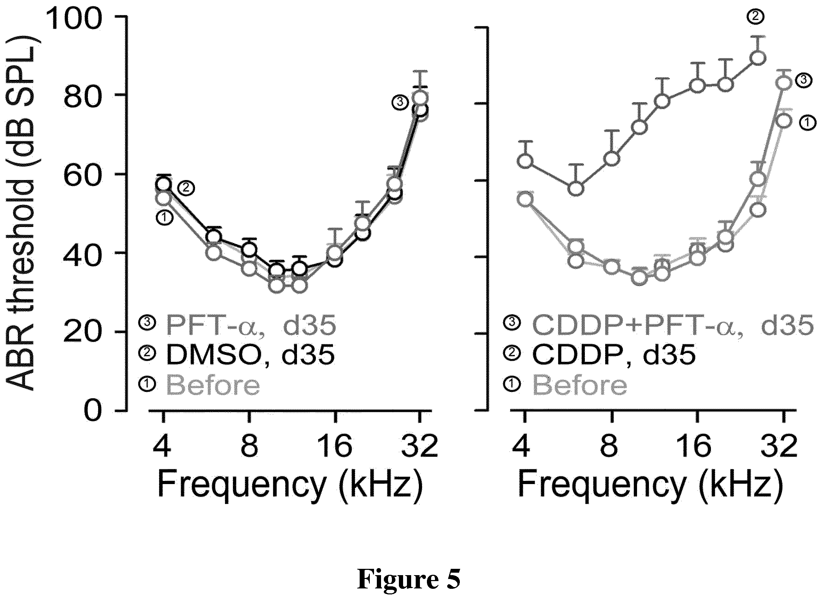

**Figure 5****:** ABR thresholds recorded prior to (Curve n°1-left panel and Curve n°1 right panel) or at 35^{th} day of DMSO-treated (Curve n°2 - left panel), PFT-α-treated (Curve n°3 - left panel), CDDP-treated (Curve n°2 - right panel) or CDDP+ PFT-α-treated (Curve n°3 - right panel) mice. Abscissa: Frequency, as expressed in kHz. Ordinate: ABR threshold, as expressed in dB SPL.

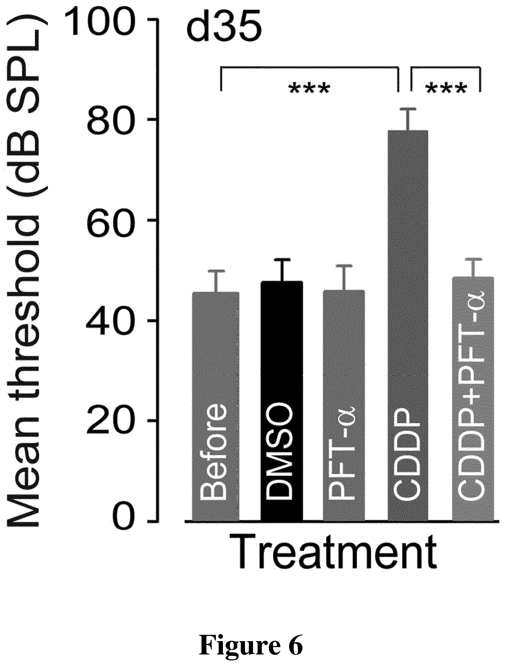

**Figure 6****:** Mean ABR threshold from 4 kHz to 32 kHz derived from the results depicted in Figure 5. Abscissa ; from left to right of the figure, Before treatment, and 35 days after DMSO, PFT-alpha, CDDP, combination of CDDP and PFT-alpha treatments, respectively. Ordinate: Mean threshold values, as expressed as dB SPL.

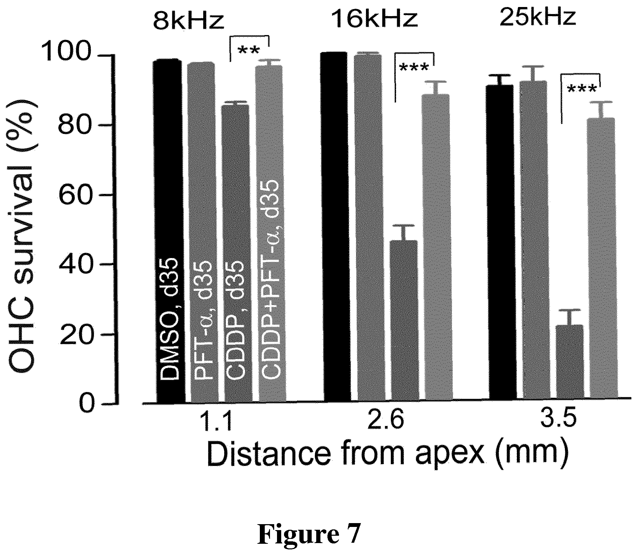

**Figure 7****:** Cytocochleograms representing the percentage of surviving hair cells in three cochlear regions located at 1.1, 2.6, and 3.5 mm from the cochlear apical end provided from 35^{th} day DMSO-treated, PFT-α-treated, CDDP-treated or CDDP+ PFT-α-treated mice. All of the data are expressed as mean ± SEM. *p<0.05, **p<0.01 and ***p<0.001. Abscissa : from left to right : distance from apex, as express in mm, for 1.1 mm, 2.6 mm and 3.5 mm, respectively. For each distance value, from left to right: results of a treatment with DMSO, PFT-alpha, CDDP, combination of CDDP and PFT-alpha, respectively. Ordinate: percentage of OHC survival.

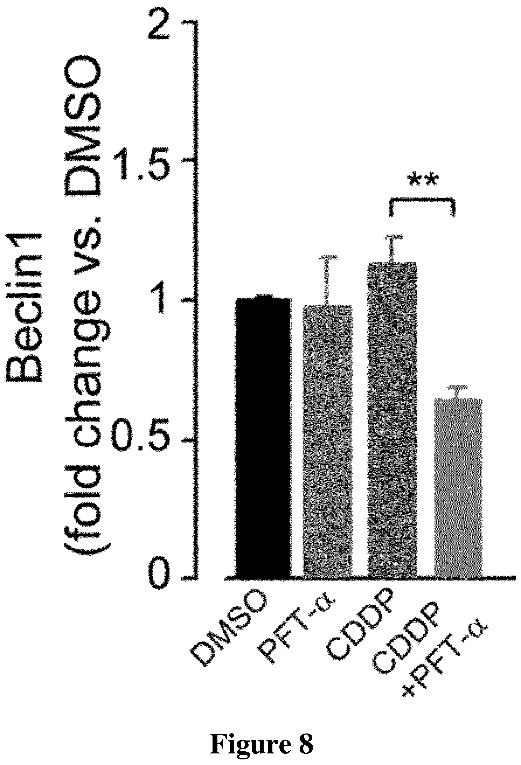

**Figure 8****:** Histograms representing the levels of Beclin 1 in HBCx-14 tumors treated with the different regimens (n = 3-4 tumors per group, all experiments were performed in triplicate). Actin served as a loading control. Data are expressed as mean ± SEM. One-way ANOVA test followed by post hoc Tukey's test (*P = 0.03, **P = 0.006, ***P ≤ 0.0004; CDDP versus DMSO or CDDP versus CDDP + PFT-α).

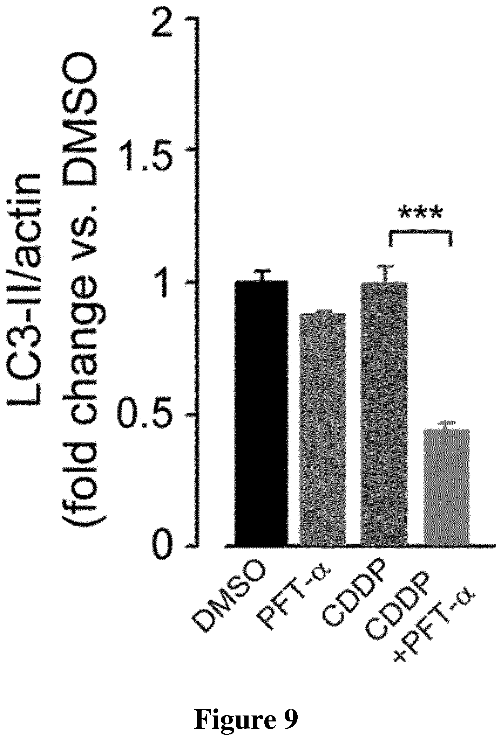

**Figure 9****:** Histograms representing the levels of LC3-II in HBCx-14 tumors treated as in Figure 8 (*P = 0.02, ***P = 0.0005; CDDP versus DMSO or CDDP versus CDDP + PFT-α).

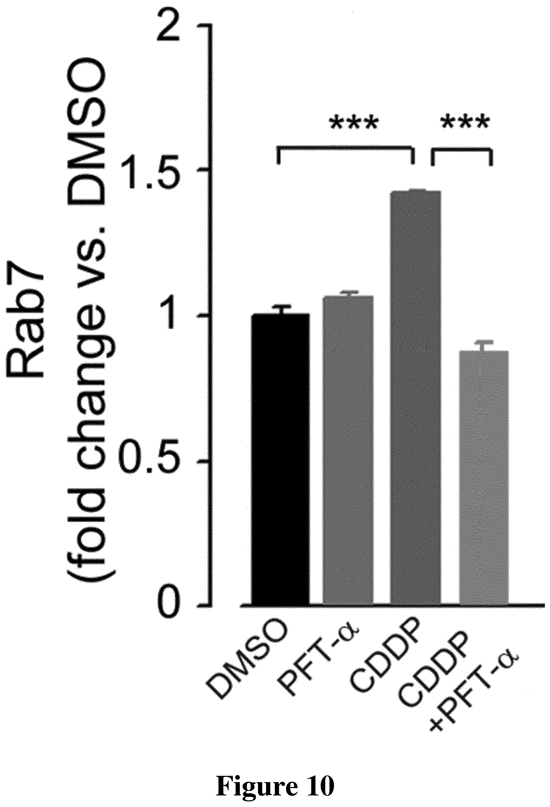

**Figure 10****:** Histograms representing the levels of Rab7 in HBCx-14 tumors treated as in Figure 8 (*P = 0.03, ***P ≤ 0.0006; CDDP versus DMSO or CDDP versus CDDP + PFT-α).

### Detailed description of the invention

The inventors have unexpectedly shown herein that a temporary inhibitor of p53 in combination with an anti-cancer treatment prevents or reduces cancer relapse in cancer individuals subjected to a cancer therapeutic treatment.

As disclosed in the examples herein, a temporary inhibitor of p53, when used in combination with an anti-cancer treatment, allows at least a substantial tumor reduction and in most cases a complete disappearance of a tumor in cancer individuals, and further the arrest of the tumor growth rate is sustained for a long period of time, whereas, most importantly, prevents tumor recurrence.

As shown in the examples herein, a temporary inhibitor of p53, when used in combination with an anti-cancer treatment, induces an almost complete loss of CD133 positive cells, which are cells recognized as consisting of cancer stem cells. Without wishing to be bound by any particular theory, the inventors believe that the loss of cancer stem cells which has been determined in the presence of a temporary inhibitor of p53 may substantially account for the effect of prevention of tumor recurrence, i.e. of tumor relapse, in cancer individuals subjected to an anti-cancer treatment. The inventors cannot exclude that one or more other unknown physiological effects induced by the presence of a temporary inhibitor of p53 may account for the effect of prevention or reduction of tumor recurrence, or may contribute to this effect of prevention or reduction of tumor recurrence.

The effect of prevention or reduction of tumor relapse by a temporary inhibitor of p53 that has been shown by the inventors is believed to improve the survival time of cancer patients.

Further, the effect of prevention or reduction of tumor relapse by a temporary inhibitor of p53 which is shown herein, when combined with a cancer therapeutic treatment, is associated with (i) a reduction of undesirable effects caused by the said cancer therapeutic treatment, e.g. a reduced cytotoxicity such as a reduced ototoxicity and (ii) a potentialisation of the anti-tumor effect provided by the said cancer therapeutic treatment. Thus, the present invention relates to a temporary inhibitor of p53 for use for preventing or reducing cancer relapse in cancer patients, wherein the temporary inhibitor of p53 is used in combination with an anti-cancer treatment, said anti-cancer treatment being a radiation or an anti-cancer agent, and wherein the temporary inhibitor of p53 is chosen from pifithrin-alpha, pifithrin -µ, and cyclic pifithrin-alpha hydrobromide.

It is known in the state of the art, antagonists of the interaction between p53 and Mdm2, useful in treating patients with relapsed/refractory acute myeloid and lymphoid leukemia and refractory chronic lymphocytic leukemia/small cell lymphocytic lymphomas (WO 2012/033525). However, said antagonists are capable of restoring wild type p53 activity rather than being inhibitors of p53 activity.

As used herein, "a temporary inhibitor of p53" refers to an inhibitor of p53 that is capable of transiently, transitorily and non-permanently promoting inhibition of p53 expression and/or activity after administration.

In other words, the "temporary" inhibitor performs its inhibitory activity for a determined time period.

As used herein, a "cancer patient" means a human patient affected with a cancer disease.

The term "prevent" refers to any success or indicia of success in the forestalling of cancer recurrence/relapse in patients in clinical remission, which includes breast cancer recurrence/relapse in patients in clinical remission, as measured by any objective or subjective parameter, including the results of a radiological or physical examination.

The terms "cancer recurrence" and "cancer relapse" may be used interchangeably herein, and refer to the diagnosis of return, which includes radiographic diagnosis of return, or signs and symptoms of return of cancer, notably breast cancer after a period of improvement or remission.

As used herein, "treatment" refers to treatment producing a beneficial effect, e.g., amelioration of at least one symptom of a disease or disorder. A beneficial effect can take the form of an improvement over baseline, i.e., an improvement over a measurement or observation made prior to initiation of therapy according to the method. A beneficial effect can also take the form of arresting, preventing, eradicating, reducing, slowing, retarding, or stabilizing of a deleterious progression of a marker of a cancer disease. For example, treatment may refer to reducing the number of cancer stem cells. Treatment may also refer to inhibiting growth of CD133 positive cells or eliminating CD133 positive cells.

As used herein a cancer disease encompass cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

As used herein, "anti-cancer treatment" encompasses therapeutic treatment of cancer by radiation and therapeutic treatment of cancer by administration of anti-cancer agents.

As used herein, "anti-cancer agent" refers to any agent that has the functional property of inhibiting the proliferation of tumor cells and of inhibiting the development or progression of a cancer disease.

The description also discloses, not according to the invention, a temporary inhibitor of p53 for its use for eliminating cancer stem cells in cancer patients, especially in patients subjected to an anti-cancer treatment. The use of a temporary inhibitor of p53 for preparing a medicament for eliminating cancer stem cells in cancer patients, not according to the invention, is described. The description discloses, not according to the invention, a method for eliminating cancer stem cells in cancer patients comprising a step of administering, to a patient in need thereof, a temporary inhibitor of p53. The said cancer patients may be subjected to an anti-cancer treatment.

As used herein, "cancer stem cells" refer to cancer stem cells that possess characteristics associated with normal stem cells For example, the ability to give rise to all cell types found in a particular cancer sample. Cancer stem cells are tumorigenic (tumor-forming) cells. For instance, cancer stem cells can generate tumors through the stem cell processes of self -renewal and differentiation into multiple cell types. For instance, such cells can persist in tumors as a distinct population and cause relapse and metastasis by giving rise to new tumors. Cancer stem cells encompass CD133 positive cells, as shown in the examples herein.

The description discloses, not according to the invention, the use of a temporary inhibitor of p53 for improving the survival time of cancer patients, especially for patients subjected to a cancer therapeutic treatment. Thus, this description discloses, not according to the invention, a temporary inhibitor of p53 for its use for improving the survival time of cancer patients. The description also discloses, not according to the invention, the use of a temporary inhibitor of p53 for preparing a medicament for improving the survival time of cancer patients. The description also discloses, not according to the invention, a method for improving the survival time of cancer patients comprising a step of administering, to a patient in need thereof, a temporary inhibitor of p53, especially in a patient that is subjected to an anti-cancer therapeutic treatment.

Patients subjected to an anti-cancer treatment encompass patients subjected to an anti-cancer radiation treatment and patients that are administered with one or more anti-cancer agents.

The present invention also relates to an anti-cancer agent for use in combination with a temporary inhibitor of p53 for use for preventing or reducing cancer relapse in cancer patients, wherein the temporary inhibitor of p53 is chosen from pifithrin-alpha, pifithrin-µ, and cyclic pifithrin-alpha hydrobromide.

This description also discloses, not according to the invention, the use of an anti-cancer treatment in combination with a temporary inhibitor of p53 for reducing or eliminating cancer stem cells in cancer patients. This description also discloses, not according to the invention, the use of an anti-cancer agent for its use in combination with a temporary inhibitor of p53 for manufacturing a medicament for reducing or eliminating cancer stem cells in cancer patients. This description also discloses, not according to the invention, an anti-cancer agent for its use in combination with a temporary inhibitor of p53 for reducing or eliminating cancer stem cells in cancer patients. This description also discloses, not according to the invention, a method for reducing or eliminating cancer stem cells in cancer patients comprising a step of administering, to a patient in need thereof, an anti-cancer agent in combination with a temporary inhibitor of p53.

As used herein, the expression "reducing cancer stem cells" encompasses reducing the number of cancer stem cells in the tumor tissue, e.g. reducing the number of CD133 or CD44 positive cells in a given volume or in a given section area of the tumor tissue (in reference to the examples herein).

### Embodiments

The temporary inhibitor of p53 is administered to patients that are subjected to an anti-cancer treatment, e.g. an anti-cancer treatment by exposure of part or all of the patient's body to radiation or an anti-cancer treatment comprising administration of one or more anti-cancer agents to the said patients.

In some of these preferred embodiments, the said temporary inhibitor of p53 and the said anti-cancer agent are each separately administered to the said cancer patient.

In some of these preferred embodiments, the said temporary inhibitor of p53 is administered previously to the said anti-cancer agent.

In some of these preferred embodiments, the administration of the said temporary inhibitor of p53 to the said cancer patient is interrupted previously to stopping the administration of the said anti-cancer agent.

In some of these preferred embodiments, the said temporary inhibitor of p53 and the said anti-cancer agent are concomitantly administered to the said cancer patient.

In some of these preferred embodiments, the said temporary inhibitor of p53 and the said anti-cancer agent are combined in a single pharmaceutical composition which is administered to the said cancer patient.

A plurality of these embodiments are described hereunder.

In preferred embodiments, the temporary inhibitor of p53 is co-administered with the anti-cancer agent. According to these embodiments, the temporary inhibitor of p53 is used in co-administration with an anti-cancer agent, for preventing or reducing cancer relapse in cancer patients. The temporary inhibitor of p53 for use according to the invention may comprise the steps of (a) administering the anti-cancer agent to the said cancer patient and (b) administering the temporary inhibitor of p53 to the said cancer patient. According to these embodiments, a cancer patient is subjected both to the anti-cancer treatment and to administration of the temporary inhibitor of p53, irrespective of whether the anti-cancer treatment and the administration of the temporary inhibitor of p53 are concomitant, i.e. (i) the temporary inhibitor of p53 is administered at a time period wherein the anti-cancer treatment is given to the said patient, (ii) the temporary inhibitor of p53 is administered at a time period when the anti-cancer treatment has not yet started and (iii) the temporary inhibitor of p53 is administered at a time period when the anti-cancer treatment has already been given to the said patient and has been stopped.

In some embodiments, the temporary inhibitor of p53 is administered subsequently to the anti-cancer treatment, e.g. subsequently to the anti-cancer agent. According to these embodiments, the temporary inhibitor of p53 is not administered to the cancer patient at a time period when the said cancer patient receives the anti-cancer treatment, e.g. at a time period when the said cancer patient is administered with an anti-cancer agent. According to these embodiments, the temporary inhibitor of p53 is administered to the said patient at a time period when the said patient has been subjected to the anti-cancer treatment that has been interrupted or terminated. According to these specific embodiments, the prior anti-cancer treatment is aimed at reducing or eliminating the tumor cells or the main tumor tissue, and the subsequent administration of the temporary inhibitor of p53 is aimed at reducing or eliminating the possibly remaining cancer stem cells, especially the possibly remaining CD133 positive cells, so as to reduce or prevent cancer relapse.

In some other embodiments, the temporary inhibitor of p53 is administered prior to the anti-cancer agent. According to these other embodiments, the temporary inhibitor of p53 is not administered to the cancer patient at a time period when the said cancer patient receives the anti-cancer treatment, e.g. at a time period when the said cancer patient is administered with the anti-cancer agent. According to these other embodiments, the administration of the temporary inhibitor of p53 is aimed at reducing or eliminating the tumor cells or the main tumor tissue, and the subsequent administration of the temporary inhibitor of p53 is aimed at reducing or eliminating the possibly remaining cancer stem cells, especially the possibly remaining CD133 positive cells, so as to improve the efficiency of a subsequent anti-cancer treatment and thus reduce or prevent cancer relapse. In still other embodiments, the temporary inhibitor of p53 is administered concurrently with an anti-cancer agent. According to these embodiments, the temporary inhibitor of p53 is administered to a cancer patient at least once during a time period when the said cancer patient also receives an anti-cancer treatment, e.g. during a time period when the said cancer patient is administered with the anti-cancer agent. In some aspects of these still other embodiments, administration of a temporary inhibitor of p53 may be continued after administration of the anti-cancer agent is terminated, whether temporarily terminated or permanently terminated, so as to sustain the effect of reduction or prevention of cancer relapse.

This invention also relates to an anti-cancer agent for use use in combination with a temporary inhibitor of p53 for use use for preventing or reducing cancer relapse in cancer patients wherein the temporary inhibitor of p53 is chosen from pifithrin-alpha, pifithrin -µ, and cyclic pifithrin-alpha hydrobromide.

As disclosed above, according to these embodiments also, (i) the temporary inhibitor of p53 is administered at a time period wherein the anti-cancer treatment is given to the said patient, (ii) the temporary inhibitor of p53 is administered at a time period when the anti-cancer treatment has not yet started and (iii) the temporary inhibitor of p53 is administered at a time period when the anti-cancer treatment has already been given to the said patient and has been stopped.

### Temporary inhibitor of p53

As used herein, a temporary inhibitor of p53 encompasses agents that cause temporary or reversible inhibition of p53 activity and wherein the said temporary or reversible inhibition of p53 activity ceases in a short time period subsequent to the interruption of the administration thereof to a patient, e.g. in a time period of 12 hours or more subsequent to the interruption of the administration thereof to a patient. In this specific context, a time period of 12 hours or more encompasses a time period of 24 hours or more, a time period of 36 hours or more, a time period of 48 hours or more, a time period of 72 hours or more and a time period of 96 hours or more. It is herein specified that an inhibition of p53 activity encompasses an inhibition of the expression of a p53 gene and an inhibition of the production of a p53 protein. According to the present invention the "temporary inhibitor of p53" is chosen from pifithrin-alpha, pifithrin -µ, and cyclic pifithrin-alpha hydrobromide.

As used herein, the term "reversible" means that after a given a period of time upon administration, the inhibitor stops performing its inhibitory activity, hence reverting to the physiological functioning of the p53 signaling pathway.

As used herein, the term "temporary" and "reversible" may be employed indistinctly to refer to the inhibitor of p53.

A skill person in the art may identify the time period under which a temporary inhibitor of p53 performs its inhibitory activity.

Temporary inhibitors of p53, which include pifithrin-alpha, are for example described in the PCT application published under number WO 00/44364, which temporary inhibitors of p53 include any one of the temporary inhibitors of p53 of formula (I), (II), (III) and (IV) that are disclosed in this document. Methods for synthesizing temporary inhibitors of p53 of formula (I), (II), (III) and (IV) are disclosed in the same document. The PCT application n° WO 00/44364 stated that pifithrin-alpha behaves as an inhibitor of p53-dependent cell apoptosis. This document notably described that pifithrin-alpha suppresses the *in vitro* p53-dependent apoptosis of transformed mouse embryo fibroblasts caused by doxorubicin, etoposide, taxol, cytosine, arabinoside, UV light and gamma radiation. This document also described that pifithrin-alpha protects mice from gamma radiation-induced death. This document also described that mice treated with both cyclophosphamide and pifithrin-alpha exhibited a higher decrease in tumor growth, as compared with mice treated with cyclophosphamide alone. However, the PCT application n° WO 00/44364 is not concerned with a solution to the problem of reducing or preventing cancer relapse.

In preferred embodiments, a temporary inhibitor of p53 is pifithrin-alpha or a salt thereof, which includes hydrogen bromide of pifithrin-alpha.

For the sake of clarity, pifithrin-alpha has the following formula (A) below:

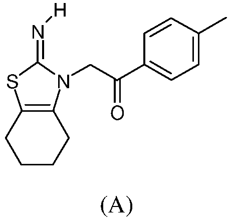

A temporary inhibitor of p53 also encompasses antisense polynucleotides targeting the human p53 gene, this type of temporary inhibitor of p53 is not part of the present invention. The human p53 gene and messenger RNA nucleic acid sequences are known in the art, as well as general methods for generating antisense polynucleotides starting from a known polynucleotide sequence. Further, a number of p53 antisense polynucleotides have been described in the art, which include those described by Mahmoudi et al. (2009, Mol Cell, Vol. 33(4) ; 462-471); Hirota et al. (1996, Japan J Cancer Res, Vol. 87(7) ; 735-742), Matsushita et al. (2000, Circulation, Vol. 101 : 1447-1452), Sak et al., (2003, Cancer Gene Ther 10: 926-934), Tepel et al. (Pancreas. 2004, 28(1):1-12) or Gorska et al. (2013, PlosOne, Vol. 8 (11) : e78863- p 1-15).

A temporary inhibitor of p53 also encompasses antibodies directed against p53, this type of temporary inhibitor of p53 is not part of the present invention. The human p53 protein amino acid sequence is known in the art, as well as a vast number of methods for generating antibodies directed against a known polypeptide. Illustratively, it may be used an anti-p53 antibody marketed by the Company Cell Signaling and available from the Company Ozyme (France) under the reference #9282. For anti-p53 antibodies, the one skilled in the art may also refer to those described by and by Wang et al. (2010, Translational Oncology, Vol. 3 (1): 1-12).

In some embodiments, the present invention involves the administration of 0.001 mg/kg to 200 mg/kg of the temporary inhibitor of p53 in a single dosage form suitable for oral sublingual, intravenous, intraperitoneal subcutaneous or intramuscular administration.

Multiple doses often are desired, or required, because the suppression of p53 activity is temporary
In some embodiments, the present invention involves the administration of 0.001 mg/kg to 200 mg/kg per day of pifithrin-alpha in a single dosage form or as separate dosage forms suitable for oral sublingual, intravenous, intraperitoneal subcutaneous or intramuscular administration. Multiple doses often are desired, or required, because the suppression of p53 activity is temporary.

Preferably, the temporary inhibitor of p53, and especially of pifithrin-alpha, is for use for administration to a human individual in need thereof.

Administration of the temporary inhibitor of p53, and especially of pifithrin-alpha, is part of the technical knowledge of the one skilled in the art.

### Cancer treatment

As used herein, a "cancer treatment" encompasses any kind of techniques for therapeutic treatment of a cancer disease, and especially therapeutic treatment of cancer by exposing a tumor tissue to radiation as well as therapeutic treatment of cancer by administration of one or more anti-cancer agents, i.e. one or more anti-cancer active ingredient. According to the invention, the anti-cancer treatment is a radiation or an anti-cancer agent.

### Cancer disease

As already disclosed herein, a cancer disease encompass cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor. Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small- intestine, and salivary gland cancers.

Tumors of the urinary tract include, but are not limited to bladder, penile, kidney, renal pelvis, ureter, urethral and human papillary renal cancers.

Eye cancers include, but are not limited to intraocular melanoma and retinoblastoma. Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma. Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal, hypopharyngeal, nasopharyngeal, oropharyngeal cancer, lip and oral cavity cancer and squamous cell. Lymphomas include, but are not limited to AIDS- related lymphoma, non-Hodgkin's lymphoma, cutaneous T-cell lymphoma, Burkitt lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia. These disorders have been well characterized in humans, but also exist with a similar etiology in other mammals, and can be treated by administering pharmaceutical compositions of the present invention.

In specific embodiments, the cancer disease consists of a breast cancer.

It is known in the art that a number of cancer treatment techniques, which includes treatment by exposure to radiation as well as treatment by administration of anti-cancer agents, cause an activation of p53 expression in tumor stem cell like cells, tumor surrounding cells and in the cells of normal tissues.

### Cancer treatment by exposure to radiation

Radiation therapy is a standard treatment for controlling unresectable or inoperable tumors and/or tumor metastases. Improved results have been seen when radiation therapy has been combined with other anti-cancer therapy. Radiation therapy is based on the principle that high-dose radiation delivered to a target area will result in the death of reproductive cells in both tumor and normal tissues. The radiation dosage regimen is generally defined in terms of radiation absorbed dose (Gy), time and fractionation, and must be carefully defined by the oncologist. The amount of radiation a patient receives will depend on various considerations, but the two most important are the location of the tumor in relation to other critical structures or organs of the body, and the extent to which the tumor has spread. A typical course of treatment for a patient undergoing radiation therapy will be a treatment schedule over a 1 to 6 week period, with a total dose of between 10 and 80 Gy administered to the patient in a single daily fraction of about 1.8 to 2.0 Gy, 5 days a week. In a preferred embodiment of this invention there is synergy when tumors in human patients are treated with the combination treatment of the invention. In other words, the inhibition of tumor growth by means of the radiation treatment of the combination of this invention is enhanced when combined with treatment using an anti-cancer agent. Parameters of adjuvant radiation therapies are, for example, contained in International Patent Publication WO 99/60023. Further details of the methodology of radiation treatment of cancer patients is well known to those of skill in the art, and is readily available from the extensive literature in this area (e.g. Principles and Practice of Radiation Oncology (2003), 4th Edition, ISBN 0-7817-35254, ed. Perez C. A. et al., Lippincott Williams and Wilkins; Radiotherapy for head and Neck Cancers (2002), 2nd Edition, ISBN 0-7817-2650-6, Ang, K. K. and Garden, A. S., Lippincott Williams and Wilkins; Principles and Practice of Oncology (2001), 6th Edition, ISBN 0-7817-2387-6, ed. DeVita, V. T. et al., Lippincott Williams and Wilkins).

The source of radiation may be either external or internal to the patient being treated. When the source is external to the patient, the therapy is known as external beam radiation therapy (EBRT). When the source of radiation is internal to the patient, the treatment is called brachytherapy. Radioactive atoms for use in the context of this invention can be selected from the group including, but not limited to, radium, ¹³⁷cesium, ¹⁹²iridium, ²⁴¹americium, ¹⁹⁸gold, ⁵⁷cobalt, ⁶⁷copper, ⁹⁹technetium, ¹²³iodine, ¹³iodine, and ¹¹¹indium.

### Cancer treatment by administration of one or more anti-cancer agents

Anti-cancer agent includes with no limitation; mitotic inhibitors, such as vinblastine; alkylating agents, such as cisplatin, carboplatin and cyclophosphamide; antimetabolites, such as 5-fluorouracil, cytosine arabinoside, hydroxyurea; nucleic acid intercalating agents, such as adriamycin and bleomycin; enzymes, such as asparaginase; topoisomerase inhibitors, such as etoposide; biological response modifiers, such as interferon; apoptotic agents, such as actinomycin D; antihormones, for example antioestrogens such as tamoxifen or, for example antiandrogens; agents which increase immune response to tumors and signal transduction inhibitors. Other examples of anti-cancer agents include: heat shock protein inhibitors (17- A AG), farnesyltransferase inhibitors (zarnestra), histone deacetylase inhibitors (SAHA, depsipeptide, MS-275..), CDK inhibitors (flavopiridol), proteasome inhibitors (bortezomib), demethylating agents (decitabine, vizada), Bcl-2 inhibitors (ABT- 737), anthracyclines, daunorubicin, doxorubicin, idarubicin, cytarabine, etoposide, dexamethasone, methotrexate, thioguanine, 6-mercaptopurine, ATRA, gemcitabine, vincristine, prednisone, mitoxantrone, and rituxan.

In the context of this invention, additional other anti-cancer agents, or compounds that enhance the effects of such agents, include, for example: alkylating agents or agents with an alkylating action, such as cyclophosphamide (CTX; e.g. cytoxan^{®}), chlorambucil (CHL; e.g. leukeran^{®}), cisplatin (CisP; e.g. platinol^{®}) busulfan (e.g. myleran^{®}), melphalan, carmustine (BCNU), streptozotocin, triethylenemelamine (TEM), mitomycin C, and the like; anti-metabolites, such as methotrexate (MTX), etoposide (VP16; e.g. vepesid^{®}), 6-mercaptopurine (6MP), 6-thiocguanine (6TG), cytarabine (Ara-C), 5-fluorouracil (5-FU), capecitabine (e.g.Xeloda^{®}), dacarbazine (DTIC), and the like; antibiotics, such as actinomycin D, doxorubicin (DXR; e.g. adriamycin^{®}), daunorubicin (daunomycin), bleomycin, mithramycin and the like; alkaloids, such as vinca alkaloids such as vincristine (VCR), vinblastine, and the like; and other antitumor agents, such as paclitaxel (e.g. taxol^{®}) and pactitaxel derivatives, the cytostatic agents, glucocorticoids such as dexamethasone (DEX; e.g. decadron^{®}) and corticosteroids such as prednisone, nucleoside enzyme inhibitors such as hydroxyurea, amino acid depleting enzymes such as asparaginase, leucovorin, folinic acid, raltitrexed, and other folic acid derivatives, and similar, diverse antitumor agents. The following agents may also be used as additional agents: amifostine (e.g. ethyol^{®}), dactinomycin, mechlorethamine (nitrogen mustard), streptozocin, cyclophosphamide, lomustine (CCNU), doxorubicin lipo (e.g. doxil^{®}), gemcitabine (e.g. gemzar^{®}), daunorubicin lipo (e.g. daunoxome^{®}), procarbazine, mitomycin, docetaxel (e.g. taxotere^{®}), aldesleukin, carboplatin, oxaliplatin, cladribine, camptothecin, CPT 11 (irinotecan), 10-hydroxy 7-ethyl-camptothecin (SN38), floxuridine, fludarabine, ifosfamide, idarubicin, mesna, interferon alpha, interferon beta, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, and chlorambucil. In some embodiments, an anti-cancer agent is selected in a group consisting of anti-cancer therapeutic antibodies.

In some embodiments, an anticancer agent is selected in a group consisting of anti-cancer polynucleotides, which includes antisens polynucleotides and small interference polynucleotides (e.g. SiRNAs).

In specific embodiments, an anti-cancer agent is cisplatin or a derivative thereof. Cisplatin may also be termed CDDP (cis-diamminedichloroplatinum(II)) herein.

Cisplatin is a platinum derivative used to treat various types of cancers, including sarcomas, some carcinomas (e.g. small cell lung cancer, and ovarian cancer), lymphomas, and germ cell tumors. It was the first member of a class of anti-cancer drugs which now also includes carboplatin and oxaliplatin. These platinum complexes react in vivo, binding to and causing crosslinking of DNA which ultimately triggers apoptosis (programmed cell death).

Cisplatin (CDDP), approved for clinical treatment by the Food and Drug Administration (FDA) in 1978, belongs to a family of platinum-containing compounds used in the treatment of various types of cancers such as bladder, ovarian, and testicular cancers. While effective as an antitumor agent, its application is limited due to toxic side effects; especially nephrotoxicity. As a consequent of the limitations of cisplatin, numerous analogues have been developed and studied with the goal of finding compounds that could be more effective and possess less toxicity. In 1989, carboplatin was the first cisplatin analogue approved by the FDA for the treatment of lung and ovarian cancer. Oxaliplatin became the third platinum (Pt)-containing compound approved by the FDA in 2002 for the treatment of colorectal cancer.

United States patent No. US 4,177,263 describes various methods for utilizing cisplatin and cisplatin analogs of Pt(II) or Pt(IV) containing various amine and chloro groups to treat tumor cells. United States patent No. US 4,584,316 describes the synthesis and use of palladium complexes as anti-tumor agents. Cisplatin (cis-diamminedichloroplatinum, cis-Pt(NH₃)₂Cl₂) has been used as a chemotherapeutic agent for many years since the discovery of its anti-tumor activity by B. Rosenberg et. al. (Nature, 1965, 205, 698; Nature, 1972, 222, 385). Chemical & Engineering News (Oct. 23, 1995). In 1979, it was approved by the Food and Drug Administration for clinical treatment of testicular and ovarian tumors and cancers of the head and neck. Cisplatin and an analog, carboplatin, are now among the most widely used anticancer drugs."

In some embodiments of the invention, the said anticancer agent is a platinum anticancer drug, preferably selected in a group comprising cisplatin, carboplatin, oxaliplatin, nédaplatin, satraplatin, picoplatin, phenanthriplatin, triplatin, lipoplatin, Pt(ESDT)(Py)Cl., Methotrexate, Doxorubicin, Daunorubicin, Temozolomide (TMZ) and Chloroethylating nitrosoureas. In some embodiments of the invention, the said anticancer agent is cisplatin or a cisplatin derivative or a cisplatin complex.

In some embodiments, the present invention involves the administration of 1mg/m² to 1000 mg/m² of body surface area per unit dosage form of an anti-cancer agent in a single dosage form or as a plurality of separate dosage forms suitable for oral sublingual, intravenous, intraperitoneal subcutaneous and intramuscular administrations.

Most preferably, an anti-cancer agent, including a pharmaceutical composition comprising an anti-cancer agent, is adapted for its use for intraperitoneal of for intravenous administration.

In some embodiments, the present invention involves the administration of 10 mg/m² of body surface area to 150 mg/m² of body surface area of an anti-cancer agent
Administration of an anti-cancer agent is part of the technical knowledge of the one skilled in the art.

In some embodiments, a unit dosage of an anti-cancer agent is administered each day of a time period of one to three consecutive days.

In some embodiments, a unit dosage of an anti-cancer agent is administered to the patient, for a time period of one to three consecutive days, every two to five weeks, which encompasses every three to four weeks.

Administration of an anti-cancer agent, especially of cisplatin or a derivative thereof such as CDDP, is part of the technical knowledge of the one skilled in the art.

### Compositions

In some embodiments, the temporary inhibitor of p53 and the anti-cancer agent are combined in a single pharmaceutical composition which is administered to the cancer patient.

The combined preparation comprises an effective amount of a temporary inhibitor of p53 and of an anti-cancer agent.

As used herein, "effective amount" refers to an amount of one or more agents that results in a stable cumulative incidence of response in a patient. A stable cumulative incidence of response can be achieved, for example, in the form of a stable complete remission (CR), major cytological remission (MCR) or, complete cytological remission (CCR) or, major molecular remission (MMR) or complete molecular remission (CMR) in a patient having a cancer.

The exact amount of a temporary inhibitor of p53, e.g., pifithrin-alpha, and of an anti-cancer agent, e.g. cisplatin or a derivative thereof, to be used in the combined preparation to be administered will vary according to factors such as the specific cancer cell involved, and the specific cancer disease; the degree of or involvement or the severity of the cancer disease; the size, age, and general health of the cancer patient; the response of the individual patient; the particular compound administered; the bioavailability characteristics of the preparation administered; the dose regimen selected; the kind of concurrent treatment; pharmacodynamic characteristics of the compounds and their mode and route of administration; and other relevant characteristics that the physician or as one skilled in the art, will readily determine by the use of known techniques and by observing results obtained under analogous circumstances.

In some embodiments, the present invention involves the administration of pifithrin-alpha in combination with cisplatin or of a derivative thereof. In general, doses employed for adult human treatment are in the range of 0.001 mg/kg to about 200 mg/kg per day. A preferred dose is about 1 µg/kg to about 100 µg/kg per day in combination with 50 mg/m2 to 100 mg/ m2 of cisplatin or of a derivative thereof in a single dosage form or as separate dosage forms suitable for oral sublingual, intravenous, intraperitoneal subcutaneous and intramuscular administrations. Multiple doses often are desired, or required, because the suppression of p53 activity is temporary.

In certain embodiments, the anti-cancer agent is first administered until a stable cumulative incidence of response is achieved, followed by a daily dose of a temporary inhibitor of p53 (e.g., pifithrin-alpha) until the patient achieves complete molecular remission (CMR).

Accordingly, the temporary inhibitor of p53 and the anti-cancer agent may be administered concurrently or sequentially. In certain embodiments, the temporary inhibitor of p53 and the anti-cancer agent are concurrently administered to a cancer patient, the administration being performed to the time when a complete molecular response (CMR) is observed in the patient. Simultaneous administration is generally used for patients which respond poorly to the anti-cancer agent; the temporary inhibitor of p53 is used to potentialize the effect of the anti-cancer agent

Accordingly, sequential administration generally may comprise a first-line therapy with the anti-cancer agent until a suitable cumulative incidence of response is achieved. The temporary inhibitor of p53 is then administered until a complete molecular response (CMR) is achieved, thereby preventing disease relapse by eliminating the residual cancer cells. Sequential administration is generally preferred when the patient is a good responder (rapid and prolonged cytological response to the anti-cancer agent).

In another aspect, the invention provides a kit for carrying out the administration of the combined preparation as defined above, comprising a temporary inhibitor of p53, as defined above and at least one anti-cancer agent, as defined above, as a single dosage form or as separate dosage forms.

The practice of the present invention employs, unless otherwise indicated, conventional techniques of cell biology, cell culture, molecular biology, transgenic biology, microbiology, recombinant DNA, and immunology, which are within the skill of the art.

The pharmaceutical unit dosage forms of a pharmaceutical composition which is used according to the invention, either (i) a pharmaceutical composition comprising a temporary inhibitor of p53, (ii) a pharmaceutical composition comprising one or more anti-cancer agents, or (iii) a pharmaceutical composition comprising a temporary inhibitor of p53 and one or more anti-cancer agents, as described herein, are optionally packaged in a packaging material and identified in print, in or on the packaging material, for use in the treatment or prevention of cancer. In some embodiments, a plurality of unit dosage forms is packaged in a packaging material and identified in print, in or on the packaging material, for use in the treatment or prevention of a cancer disease.

As used herein, a "pharmaceutical composition" refers to a preparation comprising the active ingredient(s), especially a temporary inhibitor of p53, with other chemical components, including, but not limited to, physiologically suitable carriers, excipients, lubricants, buffering agents, antibacterial agents, bulking agents (e.g. mannitol), antioxidants (e.g., ascorbic acid or sodium bisulfite), and the like.

The term "unit dosage form", as used herein, describes physically discrete units, each unit containing a predetermined quantity of one or more desired active ingredient(s) calculated to produce the desired therapeutic effect, in association with at least one pharmaceutically acceptable carrier, diluent, excipient, or combination thereof.

Herein, the phrases "physiologically acceptable carrier" and "pharmaceutically acceptable carrier", which are used interchangeably, describe a carrier or a diluent that does not cause significant irritation to the subject and does not abrogate the biological activity and properties of the active ingredient(s), especially the biological activity and properties of a temporary inhibitor of p53.

As used herein, the term "carrier" refers to a diluent, adjuvant, excipient, or vehicle with which the active ingredient(s), especially with which a temporary inhibitor of p53, is(are) administered.

Herein the term "excipient" refers to an inert substance added to a pharmaceutical composition to further facilitate administration of the active ingredient(s), especially further facilitate administration of a temporary inhibitor of p53.

A pharmaceutical composition may be formulated, for example, as sachets, pills, caplets, capsules, tablets, dragee-cores or discrete (e.g., separately packaged) units of powder, granules, or suspensions or solutions in water or non-aqueous media. Thickeners, diluents, flavorings, dispersing aids, emulsifiers or binders may be desirable.

Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose; and/or physiologically acceptable polymers such as polyvinylpyrrolidone (PVP). If desired, disintegrating agents may be added, such as crosslinked polyvinyl pyrrolidone, agar, or alginic acid or a salt thereof such as sodium alginate. Composition unit dosage forms that are used according to the present embodiments may, if desired, be presented in a pack or dispenser device, such as an FDA (the U.S. Food and Drug Administration) approved kit, which may contain one or more unit dosage forms containing the active ingredient(s), especially a temporary inhibitor of p53. The pack or dispenser device may, for example, comprise metal or plastic foil, such as, but not limited to a blister pack. The pack or dispenser device may be accompanied by instructions for administration. The pack may also be accompanied by a notice associated with the container in a form prescribed by a governmental agency regulating the manufacture, use or sale of pharmaceuticals, which notice is reflective of approval by the agency of the form of the compositions for human administration. Such notice, for example, may be of labeling approved by the U.S. Food and Drug Administration for prescription drugs or of an approved product insert.

### Examples

### Example 1 : Prevention of tumor recurrence by a combination of a temporary inhibitor of p53 and an anti-cancer agent

### A. Materials and Methods

### A.1. Animals

Neonate Swiss mice were purchased from Janvier Laboratories (Le Genest Saint Isle). Swiss nude mice bearing xenografts of a p53-Rb-PTEN triple-negative human breast cancer (HBCx-14) were purchased from the Institute Curie, Paris, France. Mice were housed in facilities accredited by the French Ministry of Agriculture and Forestry (B-34 172 36 - March 11, 2010). Experiments were carried out in accordance with the European Communities Council Directive of 24 November 1986 (86/609/EEC) regarding the care and use of animals for experimental procedures.

### A.2. Drug preparation

CDDP was purchased from Sigma. The specific inhibitor of p53 (Pifithrin-α, PFT-α) was provided by Tocris Bioscience.

For *in vivo* experiments, CDDP was freshly prepared at 0.5 mg/ml in saline and was injected intraperitoneally (IP) into the tumor-bearing Swiss nude mice at a dose of 14 mg/kg. PFT-α was dissolved in DMSO and was injected IP into the mice at a dose of 2.2 mg/kg in 0.4% DMSO. This dose was extrapolated from the previous *in vivo* studies (Guan, A., et al. Regulation of p53 by jagged1 contributes to angiotensin II-induced impairment of myocardial angiogenesis. PLoS One 8, e76529 (2013).; Liu, P., Xu, B., Cavalieri, T.A. & Hock, C.E. Pifithrin-alpha attenuates p53-mediated apoptosis and improves cardiac function in response to myocardial ischemia/reperfusion in aged rats. Shock 26, 608-614 (2006).).

### A.3. In vivo protocols

### A.3.1. P53 inhibition in patient-derived breast cancer xenograft mice

Impact of p53 inhibition on chemotherapeutic efficacy of CDDP was evaluated in a patient-derived breast cancer xenograft model (HBCx-14) (Marangoni, E., et al. A new model of patient tumor-derived breast cancer xenografts for preclinical assays. Clin Cancer Res 13, 3989-3998 (2007).). After the tumors had grown to approximately 250 to 400 mm³, mice were randomly divided into 4 groups: i) control DMSO (n=9), ii) control PFT-α (n=8), iii) CDDP (n=14), iv) CDDP+PFT-α (n=14), and the treatments were started. Control DMSO and PFT-α alone animals received 2 series of daily IP injection of DMSO (0.4%, 1ml) or PFT-α (2.2mg/kg) over 6 days at 1 week interval, respectively. The CDDP treatments consisted of two IP injections at a dose of 7 mg/kg (one on day 0, the other on day 14, Figure 1) resulting in an accumulated dose of 14 mg/kg. Each CDDP injection was preceded by IP injection of DMSO (0.4%, 1ml) 30 minutes before and then was followed by daily injection of DMSO over 5 days. CDDP plus PFT-α treatments consisted of a single injection PFT-α (2.2mg/kg) 30 min before CDDP (7mg/kg) on day 0 and day 14, that was followed by daily injection of PFT-α over 5 days.

To monitory tumor histological features, tumor angiogenesis and stem cells, 3 animals treated with PFT-α alone and 4 animals from each of the other three groups were randomly selected and sacrificed at day 21. To assess hearing function and hair cell morphologies, 5 other animals from each group had to be sacrificed at day 35 due to big tumor sizes developed in DMSO and PFT-α alone and the completely disappearance of tumors in the majority of CDDP+ PFT-α treated mice. The last 5 remaining animals from CDDP and CDDP+ PFT-α groups were kept up to day 70 for tumor recurrence evaluation.

### A.3.2. Tumor assessments and image analysis

Tumor volume was determined using digital vernier caliper measurements once per week and the formula: V = (π/6) × (d₁)² × d₂, where d₂ is the longest diameter of the tumor and d₁ is the shortest diameter of the tumor. Histological examinations of tumors were performed on 5 µm formalin-fixed paraffin sections stained with hematoxylin and eosin safranin. These sections were then scanned using a NanoZoomer (Hamamatsu Photonics, Hamamatsu City, Japan) with a 20x objective and then reviewed in a blinded fashion by a pathologist. Definiens Developer 7.1. software (Definiens, Munich, Germany) was used to estimate the ratio of fibrous scar area (only fibroelastotic stroma remaining) /tumor cell area (cellularity plus necrosis, Figure 2) for each entire tumor section (n=4 sections/tumor and 4 tumors/group).

The tumor angiogenesis and stem cell evaluations were performed on 6 µm formalin-fixed cryostat-sections. Monoclonal rat antibody against CD31 (1/150, Chemicon International) was used for tumor vascular area evaluation. Polyclonal rabbit antibody against CD133 (1:200, MyBioSource,USA) was used for detection of CD133 positive stem cell like cells (Wright, M.H., et al. Brca1 breast tumors contain distinct CD44+/CD24- and CD133+ cells with cancer stem cell characteristics. Breast Cancer Res 10, R10 (2008).). Polyclonal rabbit antibody against cytokeratin 5 was used for identification of the basal-like breast cancer cells (Martin-Castillo, B., et al. Cytokeratin 5/6 fingerprinting in HER2-positive tumors identifies a poor prognosis and trastuzumab-resistant basal-HER2 subtype of breast cancer. Oncotarget 6, 7104-7122 (2015).). The secondary antibodies used were goat anti-rat or anti-rabbit IgG conjugated to Alexa 488 or 594 (1/1000, Invitrogen, Carlsbad, USA). The Hoechst 33342 (0.002% wt:vol in water, Sigma) was used to stain DNA. Negative controls were performed by omitting the primary antibody.

Five fields at 20× microscope objective of CD31 stained sections were chosen in the most vascular part (periphery part) of the tumor to estimate the sum of area occupied by vessels, according to the conventional vascular area quantification in solid tumors (Vermeulen, P.B., et al. Second international consensus on the methodology and criteria of evaluation of angiogenesis quantification in solid human tumours. Eur J Cancer 38, 1564-1579 (2002). ; Mikalsen, L.T., et al. The clinical impact of mean vessel size and solidity in breast carcinoma patients. PLoS One 8, e75954 (2013).). The vessels were identified in the images using a user-supervised algorithm developed in the Matlab programing language (image processing toolbox, MathWorks). CD31 positive pixels were firstly automatically detected using a thresholding method (*edge* function, threshold adjusted by the user). The lumen area were then segmented using the *bwselect* function associated with the *imclose* function. In the resulting image, the profile of vessels appeared in red and its interior area (lumen area) in white (). The vascular area were then calculated using the formula: vascular area = CD31 positive area+ lumen area, only gaps with 20µm in diameter in the stains were considered to be lumens and were estimated (). The percentage of CD133 positive cells was analyzed by counting the number of positive cells per total Hoechst 33342 labelled nuclei per field in five independent fields at 20× microscope objective of CD133 stained sections. A total of 3 sections, and 4 tumors per group were used for tumor quantification.

### A.3.3. Hearing functional assessments

Auditory function was assessed by recording sound-evoked auditory brainstem responses. The recordings were performed prior to and 35 days from the beginning of CDDP treatment in patient-derived breast cancer xenograft mice. The recordings were done under anesthesia with Rompun 2% (3 mg/kg) and Zoletil 50 (40 mg/kg) in a Faraday shielded anechoic sound proof cage. Heart rate was monitored on an oscilloscope via electrocardiogram electrodes. Rectal temperature was measured with a thermistor probe, and maintained at 38.5°C ± 1°C using a heated underblanket. ABRs evoked by tone bursts (10 ms duration, 1 ms rise/fall, 10/s) were recorded using three subcutaneous needle electrodes placed on the vertex (active), on the pinna of the tested ear, and in the neck muscles (ground) of the animal. The ABR thresholds were defined as the minimum sound intensity necessary to elicit a clearly distinguishable response (> 0.2µV).

### A.3.4. Cochlear morphological assessments

Sensory hair cell loss was evaluated using scanning electron microscopy (Hitachi S4000). The cochleae from the different treatment groups (n=5 per group) were processed and evaluated using our standard technique. The hair cell counting was performed in three different 300 µm long segments of the organ of Corti, centered at 1.1, 2.6, 3.5 mm from the cochlear apical end and corresponding to the place coding of 8, 16 and 25 kHz, respectively.

### A.3.5 Biomarker analysis.

Western blotting was used for biomarker analysis. The functional integrity of the p53 pathway was analyzed for p53 and p21 in the xenograft model treated with either DMSO or CDDP. The effect of p53 inhibition on CDDP-induced DNA damage and autophagy was assessed using phosphorylated Chk-1 as DNA damage marker, and Beclin 1, LC3-II and Rab7, as autophagy markers in the xenograft model following the various treatment regimens. Western blotting analysis was performed using standard procedures. Antibodies used included those recognizing p21 (1/2,000, Cell Signaling Technology, #2946), p53 (1/1,500, Cell Signaling Technology, #2524), phospho-Chk1 (1/800, Ser345, Cell Signaling Technology, #2348), Beclin 1 (1/1,000, Santa Cruz Biotechnology, #sc11427), LC3-II (1/800, Cell Signaling Technology, #2775), Rab7 (1/800, Santa Cruz Biotechnology, #sc-376362), and β-actin (1/10,000, Sigma-Aldrich, #A1978). Secondary antibodies used were goat anti-mouse IgG (1/3,000, Jackson ImmunoResearch, #115-001-003) and goat anti-rabbit IgG (1/3,000, Jackson ImmunoResearch, #111-001-003). Western blot analysis required 3-4 tumors per group. All experiments were performed in triplicate.

### A.4. Statistics

Data are expressed as mean ± SEM. If conditions for a parametric test were met, the significance of the group differences was assessed with a one-way ANOVA; once the significance of the group differences (P < 0.05) was established, Tukey's *post hoc* tests were subsequently used for pairwise comparisons. If not, Kruskal-Wallis tests were used to assess the significance of differences among several groups; if the group differences were significant (P < 0.05), Dunn's tests were then used for *post hoc* comparisons between pairs of groups. Data analysis was performed using Matlab (The Mathworks Company). For *in vivo* mouse studies, based on data from our previous reports (Wang *et al,* 2003a; Wang *et al,* 2004) or from preliminary experiments, we calculated the sample size using G*Power 3.1.9.2 to ensure adequate power of key experiments for detecting pre-specified effect sizes.

### B. Results

Having identified p53 as a key determinant of the ototoxicity side effects of CDDP, we explore the feasibility of reversible p53 suppression in human cancer therapy. To this end, we used Swiss nude mice bearing xenografts of a p53-Rb-PTEN triple-negative human breast cancer with mutant (HBCx-14) (Marangoni *et al,* 2007) status.

One important end point for evaluating *in vivo* anti-tumor efficacy is the tumor growth inhibitory effect over a long period of time. While DMSO (1.) or PFT-α alone (2.) treated group showed continuous tumor growth over a period of 5 weeks, the CDDP-treated group (3.) showed significant tumor shrinkage (Figure 1). Surprisingly, combination of CDDP with PFT-α (4.) demonstrated a larger response to treatments as shown by the complete disappearance of tumor in the majority of mice (8 out of 10 treated-animals) and the massive tumor shrinkage in the two remaining animals at d35 (Figure 1). In the group of animals treated with the combination of drugs the arrest of the tumor growth rate was sustained for up to 8 weeks (d70) after the last dose was administered. This is a far better result than in the CDDP alone group, where tumors started to regrow just 3 weeks after the end of the treatment (d35, Figure 1).

### In vivo evaluation of tumor histological features, angiogenesis and stem cell-like cells

The histological examination of the tumors collected 1 week after the end of the treatment (d21, Figure 2) revealed that combined CDDP plus PFT-α was more efficient in replacing tumor cells by fibrous scar (Figure 2). One mechanism that may account for the tumor sensitization to the CDDP/PFT-α treatment is an alteration in the tumor angiogenesis. To probe this hypothesis, the tumor angiogenesis was evaluated in the most vascular part of the CD31 stained tumor sections. According to this scenario, combined treatments were more efficient in decreasing the vascular area in the periphery of tumors at 1 week after the stop of the treatment when compared to CDDP alone group (d21, Figure 3). These data indicate that combination of CDDP and PFT-α treatments are able to prevent tumor growth by inhibiting angiogenesis.

Since combined treatments dramatically inhibited the tumor regrowth, we hypothesized that CDDP plus PFT-α might be more effective in eliminating cancer stem-like cells that have been postulated to account for tumor recurrence ( Visvader, J.E., and G.J. Lindeman. 2012. Cancer stem cells: current status and evolving complexities. Cell Stem Cell 10:717-728). Thus, we evaluated the percentage of CD133 positive cancer cells, previously validated as a cancer stem-like cell marker in breast cancer (Croker, A.K., D. Goodale, J. Chu, C. Postenka, B.D. Hedley, D.A. Hess, and A.L. Allan. 2009. High aldehyde dehydrogenase and expression of cancer stem cell markers selects for breast cancer cells with enhanced malignant and metastatic ability. J Cell Mol Med 13:2236-2252.; Aomatsu, N., M. Yashiro, S. Kashiwagi, T. Takashima, T. Ishikawa, M. Ohsawa, K. Wakasa, and K. Hirakawa. 2012. CD133 is a useful surrogate marker for predicting chemosensitivity to neoadjuvant chemotherapy in breast cancer. PLoS One 7:e45865.) . As shown in Figure 4, combined treatments resulted in an almost complete loss of CD133 positive cells. Collectively, these data show that the stronger antitumor effect of combined treatments resulted from a larger sensitization of tumor endothelial cells and CD133+ cancer stem cells to CDDP.

Finally, we monitored hearing function in these mice. As expected, mice receiving DMSO or PFT-α did not develop hearing loss and hair cell damage (Figures 5, 6 and 7). In contrast, the CDDP-treated mice showed significant increase in ABR thresholds for all the frequencies tested (mean threshold: 77.7 dB ± 4.3) and hair cell loss (20.8% ± 4.5 of OHC survival in the region coding of 25 kHz, Figures 5, 6 and 7). However systemic injection of CDDP plus PFT-α preserve hearing (mean threshold: 48.4 dB ± 5.5) and hair cells (80.1% ± 4.8 OHC survival in the region coding of 25 kHz, Figures 5, 6 and 7) over a large extent. To understand how p53 inhibition potentiated the anticancer effect of CDDP in the p53 mutated cancer xenograft model, we assessed the accumulation of p53 and its downstream effector p21 in p53 mutant (HBCx-14) model. We observed that treatment of mice with CDDP resulted in both reduced stabilization of p53 and accumulation of p21 in HBCx-14 (TP53-mutant) tumors. These results suggest that the TP53-mutant HBCx-14 tumors retain some p53 residual transactivation activity as previously reported in sarcoma cell line and squamous cell carcinoma resected from the oral cavity of patients (Pospisilova et al, 2004; Perrone et al, 2010).

### Reversible p53 inhibition reduced the CDDP-induced autophagy selectively in the TP53-mutant HBCx-14 tumors

Previous studies revealed that in response to genotoxic stress, p53-deficient tumor cells may arrest in the S and G2 phases via Chk1 activation to allow time for DNA repair and that Chk1 inhibitors selectively potentiate the effects of DNA-damaging agents, such as chemotherapy or radiation, in TP53-mutated cancer cells (Zhao et al, 2002; Ma et al, 2011). Based on these findings, we tested the probability of an abrogation of CDDP-induced Chk1 activation with the CDDP + PFT-α combination in the TP53-mutant HBCx tumors. As expected, we found that CDDP led to an increase in Chk1 phosphorylation (p-Chk1) selectively in TP53-mutant HBCx-14 tumors. However, this CDDP-induced Chk1 phosphorylation was not modified with PFT-α.

Therefore, we investigated a potential cytoprotective role of autophagy as previously proposed to occur during anticancer therapy (Harhaji-Trajkovic et al, 2009; Chaachouay et al, 2011). We found that TP53-mutant tumors displayed a basal and drug-induced (PFT-α alone or CDDP alone) levels of Beclin 1, a major player in the autophagic initiation process (Cao & Klionsky, 2007) (Fig 8). In addition, combination of PFT-α and CDDP significantly attenuated Beclin 1 expression selectively in the TP53-mutant tumors (Fig 8). Similar results were obtained in the tumor model with Western blotting analysis for LC3-II (Fig 9), an autophagosomal marker, and Rab7 (Fig 10), a small GTP-binding protein that has a role in maturation of late autophagic vacuoles (Jager et al, 2004). Taken together, our results suggest that selective suppression of autophagy in TP53-mutant tumors by the combination of CDDP and PFT-α may at least in part account for the observed enhanced effect.

### Discussion of the results

The data presented here, provide evidence that systemic administration of CDDP plus PFT-α efficiently enhances the broad anticancer efficacy of CDDP through eliminating both tumor endothelial and stem cells. In addition, this treatment is still effective in protecting inner ear against anti-drugs-induced hearing-loss.

Having established a powerful otoprotective effect of PFT we set out to test whether its administration was compatible with the therapeutic goals of CDDP anti-cancer treatment. Even though more than half of all human cancers lack functional p53 (Soussi, T. & Wiman, K.G. Shaping genetic alterations in human cancer: the p53 mutation paradigm. Cancer Cell 12, 303-312 (2007)), the statue of p53 within cancer stromal cells (endothelial cells or fibroblasts) may play an important role in tumor growth and tumor responses to cytotoxic anti-cancer agents (Bar, J., Moskovits, N. & Oren, M. Involvement of stromal p53 in tumor-stroma interactions. Semin Cell Dev Biol 21, 47-54 (2010)), in more, p53 mutations are rare in tumor-associated stromal cells (Polyak, K., Haviv, I. & Campbell, I.G. Co-evolution of tumor cells and their microenvironment. Trends Genet 25, 30-38 (2009)). Our findings point out that p53 plays a protective role in tumor endothelium under genotoxic stress condition. These results are consistent with previous reports showing that an antiangiogenic scheduling of chemotherapy was more effective in p53-null mice (Browder, T., et al. Antiangiogenic scheduling of chemotherapy improves efficacy against experimental drug-resistant cancer. Cancer Res 60, 1878-1886 (2000)) and repression of p53 in the tumor stroma sensitizes p53-deficient tumors to radiotherapy and cyclophosphamide chemotherapy (Burdelya, L.G., et al. Inhibition of p53 response in tumor stroma improves efficacy of anticancer treatment by increasing antiangiogenic effects of chemotherapy and radiotherapy in mice. Cancer Res 66, 9356-9361 (2006)). Alternatively, inhibiting p53 mediates growth arrest and DNA repair, thereby increasing the risk of mitotic catastrophe in tumor cells and/or tumor stromal cells (Bunz, F., et al. Disruption of p53 in human cancer cells alters the responses to therapeutic agents. J Clin Invest 104, 263-269 (1999); Komarova, E.A., et al. Dual effect of p53 on radiation sensitivity in vivo: p53 promotes hematopoietic injury, but protects from gastro-intestinal syndrome in mice. Oncogene 23, 3265-3271 (2004)). In this scenario, only proliferating cell populations would be affected, so that explain why inhibition of p53 does not cause collapse of vascular endothelia in normal mouse tissues to the same extent as it does in the tumor. Finally, the suppression of CD133-positive cancer stem cells with reversible inhibition of p53 that has been identified in this study might largely contribute to superior therapeutic outcome of combined treatments. This finding is in accordance with recent reports recognizing CD133 as a potent marker of tumor-initiating cells, a negative prognostic and chemoresistance factor in numerous cancers, including breast cancer (Croker, A.K., et al. High aldehyde dehydrogenase and expression of cancer stem cell markers selects for breast cancer cells with enhanced malignant and metastatic ability. J Cell Mol Med 13, 2236-2252 (2009). ; Aomatsu, N., et al. CD133 is a useful surrogate marker for predicting chemosensitivity to neoadjuvant chemotherapy in breast cancer. PLoS One 7, e45865 (2012). ; Ieni, A., Giuffre, G., Adamo, V. & Tuccari, G. Prognostic impact of CD133 immunoexpression in node-negative invasive breast carcinomas. Anticancer Res 31, 1315-1320 (2011). ; Ohlfest, J.R., et al. Immunotoxin targeting CD133(+) breast carcinoma cells. Drug Deliv Transl Res 3, 195-204 (2013).

Although the precise relationship between reversible p53 inhibition and the sensitization of the TP53-mutant cancer to CDDP needs to be fully disentangled by further in□depth investigations, our results suggest that suppression of autophagy at least in part accounts for this sensitization. Consistent with a previous report showing that p53 mutants inhibit autophagy as a function of their cytoplasmic localization (Morselli et al, 2008), we found lower levels of several autophagy-related proteins, both basal and after CDDP intoxication, in TP53 mutants than in TP53 wt. In addition, we observed a selective and efficient PFT-α induced suppression of CDDP-induced autophagy in TP53-mutant tumors. Based on these results and those of others in cancer cells showing the protective effect of autophagy activation from CDDP or 5-FU toxicity (Harhaji-Trajkovic et al, 2009; Guo et al, 2014), we suggest that the suppression of autophagy through reversible p53 inhibition in these TP53-mutant tumors may account in part for their sensitization to CDDP.

The results generated with human breast cancer xenograft model provide proof-of-concept that reversible pharmacologic suppression of p53 may protect hearing function, enhance antitumor response of CDDP and delay tumor regrowth, thus provide strong rationale for the clinical development of PFT-α targeting p53 negative or mutated cancers.

## Claims

1. A temporary inhibitor of p53 for use for preventing or reducing cancer relapse in cancer patients,
wherein the temporary inhibitor of p53 is used in combination with an anti-cancer treatment, said anti-cancer treatment being a radiation or an anti-cancer agent, and
wherein the temporary inhibitor of p53 is chosen from pifithrin-alpha, pifithrin -µ, and cyclic pifithrin-alpha hydrobromide.

2. A temporary inhibitor of p53 for use according to claim 1, wherein the said temporary inhibitor of p53 and the said anti-cancer agent are each separately administered to the said cancer patient.

3. A temporary inhibitor of p53 for use according to claim 1, wherein the said temporary inhibitor of p53 is administered previously to the said anti-cancer agent.

4. A temporary inhibitor of p53 for use according to claim 1, wherein the administration of the said temporary inhibitor of p53 to the said cancer patient is interrupted previously to stopping the administration of the said anti-cancer agent.

5. A temporary inhibitor of p53 for use according to claim 1, wherein the said temporary inhibitor of p53 and the said anti-cancer agent are concomitantly administered to the said cancer patient.

6. A temporary inhibitor of p53 for use according to claim 1, wherein the said temporary inhibitor of p53 and the said anti-cancer agent are combined in a single pharmaceutical composition which is administered to the said cancer patient.

7. An anti-cancer agent for use in combination with a temporary inhibitor of p53 for use for preventing or reducing cancer relapse in cancer patients,
wherein the temporary inhibitor of p53 is chosen from pifithrin-alpha, pifithrin -µ, and cyclic pifithrin-alpha hydrobromide.

8. A temporary inhibitor of p53 for its use according to any one of claims 1 to 6, or an anticancer agent for its use according to claim 7 wherein the said anticancer agent is selected in a group comprising cisplatin, carboplatin, oxaliplatie, nédaplatin, satraplatin, picoplatin, phenanthriplatin, triplatin, lipoplatin, Pt(ESDT)(Py)Cl, Methotrexate, Doxorubicin, Daunorubicin, Temozolomide (TMZ) and Chloroethylating nitrosoureas.

9. A temporary inhibitor of p53 for its use according to any one of claims 1 to 6, or an anticancer agent for its use according to claim 7, wherein the said anticancer agent is cisplatin or a cisplatin derivative or a cisplatin complex or a DNA-damaging compound.

10. A temporary inhibitor of p53 for use according to any one of claims 1 to 6, or an anticancer agent for use according to claim 7, wherein the said cancer patients are affected with breast cancer.

## Patentansprüche

1. Temporärer Hemmer von p53 zur Verwendung bei der Prävention oder Verringerung eines Krebsrezidivs bei Krebspatienten,
wobei der temporäre Hemmer von p53 in Kombination mit einer Antikrebsbehandlung verwendet wird, wobei die Antikrebsbehandlung eine Bestrahlung oder ein Antikrebsmittel ist, und
wobei der temporäre Hemmer von p53 aus Pifithrin-alpha, Pifithrin-µ und cyclischem Pifithrin-alpha-hydrobromid ausgewählt ist.

2. Temporärer Hemmer von p53 zur Verwendung nach Anspruch 1, wobei der temporäre Hemmer von p53 und das Antikrebsmittel dem Krebspatienten jeweils getrennt verabreicht werden.

3. Temporärer Hemmer von p53 zur Verwendung nach Anspruch 1, wobei der temporäre Hemmer von p53 vor dem Antikrebsmittel verabreicht wird.

4. Temporärer Hemmer von p53 zur Verwendung nach Anspruch 1, wobei die Verabreichung des temporären Hemmers von p53 an den Krebspatienten vor dem Stoppen der Verabreichung des Antikrebsmittels unterbrochen wird.

5. Temporärer Hemmer von p53 zur Verwendung nach Anspruch 1, wobei der temporäre Hemmer von p53 und das Antikrebsmittel dem Krebspatienten begleitend verabreicht werden.

6. Temporärer Hemmer von p53 zur Verwendung nach Anspruch 1, wobei der temporäre Hemmer von p53 und das Antikrebsmittel in einer einzelnen pharmazeutischen Zusammensetzung kombiniert sind, die dem Krebspatienten verabreicht wird.

7. Antikrebsmittel zur Verwendung in Kombination mit einem temporären Hemmer von p53 zur Verwendung bei der Prävention oder Verringerung eines Krebsrezidivs bei Krebspatienten,
wobei der temporäre Hemmer von p53 aus Pifithrin-alpha, Pifithrin-µ und cyclischem Pifithrin-alpha-hydrobromid ausgewählt ist.

8. Temporärer Hemmer von p53 zu seiner Verwendung nach einem der Ansprüche 1 bis 6 oder Antikrebsmittel zu seiner Verwendung nach Anspruch 7, wobei das Antikrebsmittel aus einer Gruppe ausgewählt ist, die Cisplatin, Carboplatin, Oxaliplatie, Nédaplatin, Satraplatin, Picoplatin, Phenanthriplatin, Triplatin, Lipoplatin, Pt(ESDT)(Py)Cl, Methotrexat, Doxorubicin, Daunorubicin, Temozolomid (TMZ) und chlorethylierende Nitrosoharnstoffe umfasst.

9. Temporärer Hemmer von p53 zu seiner Verwendung nach einem der Ansprüche 1 bis 6 oder Antikrebsmittel zu seiner Verwendung nach Anspruch 7, wobei das Antikrebsmittel Cisplatin oder ein Cisplatinderivat oder ein Cisplatinkomplex oder eine DNA-schädigende Verbindung ist.

10. Temporärer Hemmer von p53 zur Verwendung nach einem der Ansprüche 1 bis 6 oder Antikrebsmittel zur Verwendung nach Anspruch 7, wobei die Krebspatienten von Brustkrebs betroffen sind.

## Revendications

1. Inhibiteur temporaire de p53 pour son utilisation pour prévenir ou réduire les rechutes chez les patients atteints de cancer, dans laquelle l'inhibiteur temporaire de p53 est utilisé en combinaison avec un traitement anticancéreux, ledit traitement anticancéreux étant un rayonnement ou un agent anticancéreux, et dans lequel l'inhibiteur temporaire de p53 est choisi parmi la pifithrine-alpha, la pifithrine -µ et l'hydrobromide cyclique de pifithrine-alpha.

2. Inhibiteur temporaire de p53 pour son utilisation selon la revendication 1, dans laquelle ledit inhibiteur temporaire de p53 et ledit agent anticancéreux sont chacun administrés séparément au patient cancéreux.

3. Inhibiteur temporaire de p53 pour son utilisation selon la revendication 1, dans laquelle ledit inhibiteur temporaire de p53 est administré avant ledit agent anticancéreux.

4. Inhibiteur temporaire de p53 pour son utilisation selon la revendication 1, dans laquelle l'administration dudit inhibiteur temporaire de p53 au patient cancéreux est interrompue avant l'arrêt de l'administration dudit agent anticancéreux.

5. Inhibiteur temporaire de p53 pour son utilisation selon la revendication 1, dans laquelle ledit inhibiteur temporaire de p53 et ledit agent anticancéreux sont administrés de manière concomitante au patient atteint d'un cancer.

6. Inhibiteur temporaire de p53 pour son utilisation selon la revendication 1, dans laquelle ledit inhibiteur temporaire de p53 et ledit agent anticancéreux sont combinés dans une seule composition pharmaceutique qui est administrée au patient cancéreux.

7. Agent anticancéreux pour son utilisation en combinaison avec un inhibiteur temporaire de p53 pour prévenir ou réduire les rechutes chez les patients atteints de cancer, dans laquelle l'inhibiteur temporaire de p53 est choisi parmi la pifithrine-alpha, la pifithrine -µ et l'hydrobromide cyclique de pifithrine-alpha.

8. Inhibiteur temporaire de p53 pour son utilisation selon l'une quelconque des revendications 1 à 6, ou agent anticancéreux pour son utilisation selon la revendication 7, dans laquelle ledit agent anticancéreux est choisi dans un groupe comprenant le cisplatine, carboplatine, oxaliplatie, nédaplatine, satraplatine, picoplatine, phénanthriplatine, triplatine, lipoplatine, Pt(ESDT)(Py)Cl, méthotrexate, doxorubicine, daunorubicine, témozolomide (TMZ) et nitrosourées chloroéthylantes.

9. Inhibiteur temporaire de p53 pour son utilisation selon l'une des revendications 1 à 6, ou agent anticancéreux pour son utilisation selon la revendication 7, dans laquelle ledit agent anticancéreux est le cisplatine ou un dérivé du cisplatine ou un complexe de cisplatine ou un composé endommageant l'ADN.

10. Inhibiteur temporaire de p53 pour son utilisation selon l'une des revendications 1 à 6, ou agent anticancéreux pour son utilisation selon la revendication 7, dans laquelle lesdits patients cancéreux sont atteints d'un cancer du sein.
